# EUROPEAN PATENT APPLICATION

(11) **EP 1 013 280 A1**
(43) Date of publication of application: **28.06.2000**
(21) Application number: 99955432.2
(22) Date of filing: 10.06.1999
(51) Int. Cl.: A61K 38/18

(54) **SKIN PREPARATIONS FOR EXTERNAL USE**

(30) Priority: 10.06.1998 JP 17813498
(71) Applicant: BML., Inc., Tokyo 151-0051 (JP); Teika Pharmaceutical Co., Ltd., Toyama-shi, Toyama 930-0982 (JP)
(72) Inventor: HU, Jianguo, Kawagoe-shi, Saitama 350-1101 (JP); ONO, Seiitsu, Ichihara-shi, Chiba 299-0112 (JP); AIZU, Yoshinori, Kawagoe-shi, Saitama 350-1101 (JP); SHIMIZU, Natsue, Chiba-shi, Chiba 260-0032 (JP); NAKAGAWA, Hachiro, Kawagoe-shi, Saitama 350-1101 (JP); KIMURA, Takahito, Toyama-shi, Toyama 939-8076 (JP)
(74) Representative: Goddar, Heinz J., Dr.
(86) International application number: JP9903102
(87) International publication number: WO9964058

(57) **Abstract**

Skin preparations for external use containing as the active ingredient ciliary neurotrophic factor (CNTF), in particular, CNTF originating in humans. These preparations exert excellent preventive and therapeutic effects on simple wound and skin diseases with hardly curable ulcer typified by bedsore, etc.

## Description

### Technical Field

The present invention relates to a skin preparation for external use (hereinafter referred to as a skin external-use preparation). More particularly, the present invention relates to a skin external-use preparation exhibiting preventive and therapeutic effects on wounds and skin diseases accompanied by ulcer, such as decubitus.

### Background Art

There exist a variety of skin wounds and diseases (hereinafter referred to as "skin diseases or the like"), and there are provided skin external-use preparations exhibiting therapeutic effects on such skin diseases or the like on the basis of different mechanisms.

Of various skin diseases or the like, skin diseases or the like accompanied by ulcer are known to be difficult to cure.

Decubitus (so-called "bedsore") is known as a typical inveterate skin injury accompanied by ulcer.

Decubitus is an inveterate ulcer which occurs in the skin of a patient who lies in bed over a prolonged period of time such that a portion of the body is under pressure for a prolonged period, such as the back, the waist (particularly the sacral portion or an iliac crest portion), or the heel. Decubitus has clear boundaries and bears dried necrotic mass. Decubitus starts with redness and swelling of a portion subjected to pressure, after which the portion may bear blisters and erosion, and ultimately forms ulcers. In decubitus, necrosis of tissue develops so deep that muscle, tendon, and bone may be exposed. In addition, since decubitus are inveterate, if they should occur in the bedridden elderly, they cause serious medical problems.

In view of the foregoing, an object of the present invention is to find an ingredient which is effective for the treatment of not only simple wounds but also the aforementioned skin diseases or the like which are difficult to cure and accompanied by ulcers, such as decubitus, and to provide a skin external-use preparation comprising the ingredient as an active ingredient.

### Disclosure of the Invention

In order to solve the above-described problem, the present inventors performed extensive studies from different aspects. In one such study, they noticed an unexpected phenomenon; that the percentage of decubitus-bearing patients is considerably lower in patients suffering ALS (amyotrophic lateral sclerosis), which is difficult to cure and confines patients to bed over a very long period of time, than in patients suffering other disease and staying in bed over a prolonged period of time.

ALS is a typical inveterate neuropathy and therapy thereof has not been established. High amyotrophy and decrease in muscle strength are observed with progress of ALS, and these symptoms manifest in the respiratory muscle, which ultimately causes death. ALS patients cannot take nutrition sufficiently, due to amyotrophy and bulbar palsy, and as a result the entire body of an ALC patient is emaciated, and at the terminal stage the patient suffers total motor paralysis. Thus, ALS brings about all the known conditions for causing decubitus; however, decubitus rarely occurs in ALS patients; not even up to the point of death.

The present inventors have conducted detailed analysis of skin conditions of ALS patients, and, surprisingly enough, have found that ciliary neurotrophic factor (hereinafter referred to as "CNTF") is expressed in a very large amount on the surface of the skin of ALS patients. They have also found that a skin external-use preparation comprising CNTF as an active ingredient exhibits therapeutic effect on wounds and skin diseases or the like accompanied by inveterate ulcers such as decubitus. The present invention has been accomplished on the basis of these findings.

Accordingly, the present invention provides a skin external-use preparation comprising CNTF, preferably CNTF from human, as an active ingredient (hereinafter the preparation may be referred to as "the skin external-use preparation of the present invention").

Typically, the skin external-use preparation of the present invention is a skin external-use preparation which may be embodied as a "decubitus-therapeutic preparation" for treating decubitus and as a "wound-therapeutic preparation" for treating wounds.

### Brief Description of the Drawings

Fig. 1 is an electrophoresis profile showing the purification process of a GST-CNTF fusion protein from *E. coli* which has been expressed.
Fig. 2 is an electrophoresis profile showing the results of Western blotting for studying the specificity of the obtained polyclonal antibody.
Fig. 3 is a graph showing the results of studies on proliferation of keratinocyte by use of CNTF.
Fig. 4 is a staining image of the results of studies on CNTF effects on thickening and extending of human epidermis.
Fig. 5 is a staining image of the results of studies on CNTF effects on thickening and elongation of human epidermis in a control.
Fig. 6 is an HE staining image of the skin sample of a patient who has suffered ALS over a long period of time.
Fig. 7 is a staining image of the skin sample of a patient who has suffered ALS over a long period of time, obtained by use of an anti-CNTF antibody.
Fig. 8 is a staining image of the skin sample of a patient who has suffered ALS over a long period of time, obtained by use of an anti-CNTF antibody absorbed by CNTF.
Fig. 9 is an HE staining image of the skin sample of a patient who has suffered ALS over a short period of time.
Fig. 10 is a staining image of the skin sample of a patient who has suffered ALS over a short period of time, obtained by use of an anti-CNTF antibody.
Fig. 11 is a graph showing a correlation between the period over which a patient suffers ALS and the intensity of staining by use of anti-CNTF antibody.
Fig. 12 is an HE staining image of the skin sample of a patient suffering a neuropathy other than ALS.
Fig. 13 is a staining image of the skin sample of a patient suffering a neuropathy other than ALS, obtained by use of an anti-CNTF antibody.
Fig. 14 is a staining image of the skin sample of an ALS patient who is confirmed to have decubitus, obtained by use of anti-CNTF antibody.
Fig. 15 is a staining image of the skin sample of a patient suffering a neuropathy other than ALS, who is confirmed to have decubitus, obtained by use of anti-CNTF antibody.
Fig. 16 is an electrophoresis profile showing the results of studies as to whether or not CNTF is highly expressed in the skin of an ALS patient, obtained by means of an RT-PCR method.

### Best Modes for Carrying Out the Invention

Embodiments of the present invention will next be described.

CNTF, which is incorporated into the skin external-use preparation of the present invention as an active ingredient, is a neurotrophic factor discovered in 1984 (Science 204, 1434-1436 (1979); J. Neurochem. 43, 1468-1478 (1984)). After discovery of CNTF, cDNA corresponding to CNTF was cloned by researchers in Europe and the U.S. by use of rats and rabbits, to thereby determine the structure of CNTF (Nature 342, 920-923 (1989); Science 246, 1023-1025 (1989)).

It is known that CNTF is expressed and produced predominantly in Schwann cells on the periphery, and it is elucidated that CNTF is expressed and produced in astrocytes and the like in the central nervous system (Glia 5, 25-32 (1992), Dev. Brain Res. 66, 209-219 (1992); Neuron 9, 295-305 (1992); J. Cell Biol. 115, 447-459 (1991)).

Physiological significance of CNTF is also widely studied, and it has been elucidated that CNTF is a neurotrophic factor which relates to not only motor nerve but also memory, and which is necessary for survival of nerve tissue.

For example, when the ischiatic nerve of a rat is cut, most neurons die, but administration of CNTF to the cut end of the nerve may prevent death of the neurons (Nature 345, 440-441 (1990); Science 251, 1616-1618 (1991); Proc. Nat. Acad. Sci. U.S.A., 90, 2222-2226 (1993)). Meanwhile, it is reported that administration of CNTF may prevent the degenerative death of motoneuronal cells in progressive motor neuropathy of a hereditary mutant mouse (Nature 358, 502-504 (1992)). The lifetime of cultured neurons may be remarkably prolonged in the presence of CNTF (Neurosci. lett. 105, 316-320 (1989); J. Neuroscience 10, 3507-3515 (1990)). Furthermore, CNTF relates to higher mental functions such as memory, and CNTF is considered to be necessary for survival of cholinergic nerves in the hippocampal region of the brain, which is known to have strong tendency to fail in the case of dementia (J. Neuroscience 11, 3124-3134 (1991); Neuron 8, 145-158 (1992)). In addition, it is becoming apparent that CNTF may prolong the lifetime of other neurons and promote differentiation thereof (Neuron 8, 737-744 (1992); J. Cell Biol. 108, 1807-1816 (1989); Nature 335, 70-73 (1988)).

In neural regions, use of CNTF exhibiting the above-described excellent properties has been attempted in treatment of various neuropathies (J. Neurochem. 57, 1003-1012 (1991); Gene 102, 271-276 (1991); Eur. J. Biochem. 201, 289-294 (1991); Biochem. Biophys. Acta 1090, 70-80 (1991)).

However, no case has been reported in which CNTF is used as an active ingredient in a skin external-use preparation as in the case of the present invention.

CNTF which is used as an active ingredient in the skin external-use preparation of the present invention may be CNTFs of human derivation, as matter of course, or may be CNTFs derived from other animals, such as mouse, rat, and rabbit. (No particular limitation is imposed on the type of animals.) However, in consideration that the skin external-use preparation of the present invention is mainly applied to humans, human-derived CNTF is preferably used as an active ingredient in the skin external-use preparation. (When the skin external-use preparation is applied to an animal other than a human, in principle, CNTF derived from the animal is preferably used as an active ingredient.)

The nucleotide sequence of a CNTF gene has already been determined; for example, the nucleotide sequence of human-derived CNTF has been determined by McDonald et al. (described in EMBL Data Library: X60542), and desired types of CNTF gene may be obtained through a conventional method.

Namely, a supply source containing a desired gene; for example, a gene library of human neuron, may be prepared, and a clone containing a CNTF gene may be screened from the gene library, to thereby obtain the CNTF gene.

Generally, a gene library may be prepared by means of known methods. For example, human neurons are destructed under mild conditions, and the thus-destructed cells are subjected to sucrose density gradient centrifugation or cesium chloride density gradient centrifugation, to thereby isolate genomic DNA. Subsequently, the isolated genomic DNA is treated with an appropriate type-II restriction enzyme to thereby obtain a DNA fragment. The fragment is incorporated into a gene-introduction vector in accordance with the terminal sequence of the fragment, and the resultant vector is introduced into a host suitably chosen in accordance with the vector, to thereby prepare a desired gene library.

No limitation is imposed on the type of gene-introduction vector, and examples thereof include a plasmid such as pBluescript, pUC18, pBR322, pBGP120, pPCφ1, pPCφ2, pPCφ3, pMC1403, pLG200, pLG300, or pLG400; and a λ phage such as λgt10 or λZAPII.

Gene libraries as described above may alternatively be constructed by use of a commercially available kit for preparing a gene library.

Preferably, clones in the thus-prepared gene library of human neurons and the like are subjected to screening, to thereby select, to some extent, clones containing a CNTF gene. For example, DNA fragments based on the known nucleotide sequence of CNTF gene may be synthesized, and the thus-synthesized DNA fragments may be labeled and used as a probe, to thereby select clones containing a CNTF gene.

Alternatively, genomic DNA of human neuron and the like may be subjected to gene amplification, such as PCR, employing the aforementioned DNA fragment as a primer, and the thus-amplified gene may be labeled and used as a probe, to thereby select clones containing a CNTF gene.

The above-prepared labeled probe may be hybridized with a replica of a gene library of human neuron and the like, to thereby select clones which hybridize with the labeled probe. The thus-selected clones may be clones containing a CNTF gene.

Whether or not a desired CNTF gene is present in the thus-prepared clones may be confirmed through a generally known sequencing means.

For example, whether or not a desired CNTF gene is present may be confirmed by means of the Maxam-Gilbert method (Maxam, A. M., and Gilbert W., Proc. Natl. Acad. Sci. U.S.A., 74, 560 (1977)), the genomic sequence method (Church, G. M. and Gilbert W., Proc. Natl. Acad. Sci. U.S.A., 81, 1991 (1984)), the multiplex method (Church, G. M. and Kieffer-Higgins, S., Science, 240, 185 (1988)), the cycle sequence method (Murray, V., Nucleic Acids Res., 17, 8889 (1989)), the dideoxy method (Sanger, F., et al., Proc. Natl. Acad. Sci. U.S.A., 74, 5463 (1977)), or a like method.

Also, whether or not the above-described CNTF gene is present may be confirmed by use of a nucleotide sequence automatic analyzer employing the principle of the above-described means.

A desired CNTF gene may be isolated from the thus-obtained clones containing a CNTF gene.

In addition, on the basis of the nucleotide sequence of a known CNTF gene, genomic DNA of human neuron or the like may be directly subjected to a gene amplification means such as PCR, to thereby obtain a desired CNTF gene conveniently and rapidly.

Briefly, a large amount of CNTF gene may be obtained by amplification performed by means of a gene amplification method typified by PCR, employing genomic DNA of human neuron or the like as a template and a DNA fragment containing 5'-end and 3'-end sequences of CNTF gene as a primer.

Alternatively, a CNTF gene may be chemically synthesized by means of a method known *per se* such as the phosphite-triester method (Ikehara, M., et al., Proc. Natl. Acad. Sci. U.S.A., 81, 5956 (1984)), and a CNTF gene may also be synthesized by use of a DNA synthesizer employing the above synthesis method.

As described below, CNTF produced by use of the thus-obtained CNTF gene may be used as an active ingredient of the skin external-use preparation of the present invention. Furthermore, CNTF serving as an active ingredient of the skin external-use preparation encompasses a modified CNTF which are obtained by modifying the amino acid sequence of CNTF (for example, deletion, alteration, or addition of a specified amino acid or acids), so long as physiological activity of the modified CNTF is substantially the same as that of original CNTF (physiological activity of CNTF will be described below). A modified CNTF gene which is used for producing such a modified CNTF may be prepared by means of a method known *per se*.

A desired modified CNTF gene may be obtained by means of, for example, site-specific mutagenesis (Mark, D. F., et al., Proc. Natl. Acad. Sci. U.S.A., 81, 5662 (1984)) and the aforementioned chemical synthesis method of a gene.

CNTF serving as an active ingredient of the skin external-use preparation of the present invention may be produced by means of a general gene recombination technique employing a CNTF gene which can be obtained as described above. (Hereinafter, unless otherwise stated, a CNTF gene and CNTF will encompass a modified CNTF gene and modified CNTF, respectively.)

More specifically, a CNTF gene is integrated in a gene-expression vector which can express the CNTF gene, and the thus-obtained recombinant vector is introduced into a host corresponding to the properties of the vector for transformation. The resultant transformant may be cultured to thereby produce a desired CNTF.

Preferably, the gene-expression vector used herein generally contains a promoter, an enhancer, and the like in the upstream region of the gene to be expressed, and a transcription termination sequence and the like in the downstream region.

Expression of a CNTF gene may be a direct expression type or a fusion protein expression type making use of, for example, a β-galactosidase gene, a glutathione-S-transferase gene, or a thioredoxin gene.

Examples of gene-expression vectors for which *E. coli* is a suitable host include pQE, pGEX, pT7-7, pMAL, pTrxFus, pET, arid pNT26CII. Examples of gene-expression vectors for which *Bacillus subtilis* is a suitable host include pPL608, pNC3, pSM23, and pKH80.

Examples of gene-expression vectors for which yeast is a suitable host include pGT5, pDB248X, pART1, pREP1, YEp13, YRp7, and YCp50.

Examples of gene-expression vectors for which a mammalian cell or insect cell is a suitable host include p91023, pCDM8, pcDL-SRα296, pBCMGSNeo, pSV2dhfr, pSVdhfr, pAc373, pACYM1, pRc/CMV, pREP4, and pcDNA1.

In addition to these existing gene-expression vectors, other types of gene-expression vectors which are created according to needs may be used.

Introduction of a gene-expression vector containing a CNTF gene into a host cell and transformation of the host cell by the vector may be performed by means of a customary method. For example, when the host cell is *E. coli* or *Bacillus subtilis*, the calcium chloride method or the electroporation method may be used. When the host cell is a mammalian cell or an insect cell, the calcium phosphate method, the electroporation method, or the liposome method may be used.

The thus-obtained transformant may be cultured by means of a customary method, to thereby accumulate CNTF in the culture system.

Media used for the above culture may optionally be chosen in accordance with the type of selected host. For example, when the host cell is *E. coli*, LB medium or TB medium may be chosen, and when the host cell is a mammalian cell, RPMI1640 medium or a similar medium may be chosen.

Isolation and purification of CNTF from a product obtained by the above-described culture of the transformant may be performed by means of a customary method. For example, the cultured product may be subjected to any of a variety of treatment methods on the basis of physical properties and/or chemical properties of CNTF.

Specific examples of such treatment include treatment by use of a protein precipitant, ultrafiltration, gel filtration, high performance liquid chromatography, centrifugation, electrophoresis, affinity chromatography by use of a specific antibody, and dialysis. These treatments may be performed singly or in combination of two or more for isolation and purification of CNTF.

The thus-obtained CNTF may be incorporated into the skin external-use preparation of the present invention as an active ingredient.

CNTF which may be incorporated into the skin external-use preparation of the present invention does not necessarily refer to the entirety of an intact CNTF molecule which is considered a complete molecule. Thus, fragments of an intact CNTF molecule may be used so long as the fragment exhibits substantial physiological activity of CNTF.

The amount of CNTF to be incorporated in the skin external-use preparation of the present invention should be determined in accordance with specific use and dosage form of the skin external-use preparation, and is not particularly limited. However, the amount is preferably 0.001-1.0 wt.% on the basis of the entirety of the skin external-use preparation.

Thus, by incorporation of CNTF into the skin external-use preparation as an active ingredient, the preparation exhibits remarkable effects on simple wounds and skin diseases or the like accompanied by ulcer which are difficult to cure, such as decubitus.

Usage and dose of the skin external-use preparation of the present invention may optionally be chosen in accordance with specific use of the preparation and symptoms of the user, and are not particularly limited.

In addition to CNTF serving as an essential ingredient, if necessary, other pharmaceutically active ingredients may be incorporated into the skin external-use preparation of the present invention in order to make the preparation exhibit generally known pharmaceutical activities of the ingredients, so long as the ingredients do not impede the intended effect of the present invention.

For example, granulation accelerators such as solcoseryl, zildasac, isalopan, and sucrose; and antibacterial agents such as gentamicin, erythromycin, terramycin, acromycin, fradiomycin, and popidone iodine may be incorporated into the skin external-use preparation of the present invention.

The skin external-use preparation of the present invention may assume an embodiment as a wound-treatment preparation used for treating simple wounds, and particularly may assume a form of a decubitus-treatment preparation used for treating decubitus, which is difficult to cure, which embodiment represents one of best modes that exhibit remarkable effects of the skin external-use preparation.

In the case in which the skin external-use preparation of the present invention assumes a form of a wound-treating preparation, if necessary, active ingredients incorporated into conventional wound-treatment preparations; typically, the aforementioned granulation accelerators and antibacterial agents, and any other ingredients which are considered effective for treating wounds may be incorporated into the skin external-use preparation in combination with CNTF, so long as these ingredients do not impede the effect of the skin external-use preparation.

In the case in which the skin external-use preparation of the present invention assumes a form of a decubitus-treating preparation, if necessary, active ingredients incorporated into conventional decubitus-treatment preparations; for example, the aforementioned antibacterial agents and granulation accelerators, proteolytic enzyme agents, and any other ingredients which are considered effective for treating decubitus may be incorporated into the skin external-use preparation in combination with CNTF, so long as these ingredients do not impede the effect of the skin external-use preparation.

No particular limitation is imposed on the dosage form of the skin external-use preparation of the present invention, and, for example, the form may be liquid, ointment, cream, patch, and the like.

Corresponding to such dosage forms, the skin external-use preparation of the present invention may contain generally known base ingredients such as oils, surfactants, higher alcohols, preservatives, humectants, thickeners, chelating agents, colorants, perfumes, and the like.

Specific dosage forms of the skin external-use preparation of the present invention will next be described in connection with working examples.

### Examples

The present invention will next be described by way of examples, which should not be construed as limiting the invention thereto.

Unless otherwise stated, incorporation amount of an ingredient represents wt.% on the basis of the entirety of a target preparation in which the ingredient is incorporated.

### 〈Reference Example〉

### (1) Preparation of CNTF

cDNA of CNTF was prepared by means of a customary method and integrated into an *E. coli* expression vector (pGEX-2T: product of Pharmacia). The vector was introduced into *E. coli* (JM109: product of TOYOBO) and under induction effected by isopropyl-beta-D-thiogalactopyranoside (IPTG), a CNTF protein (a fusion protein with glutathione S-transferase (GST)) was expressed. The GST-fused CNTF protein was purified in one step by use of a glutathione-sepharose column (product of Pharmacia), and the thus-purified GST-fused CNTF protein was treated with thrombin. After the protein was cut to separate the GST portion and the CNTF portion, thrombin was removed by use of a Benzamide Sepharose 6B column (product of Pharmacia Biotech).

The thrombin-treated product was added to a glutathione-sepharose column again, and fragments which passed through the column without binding thereto were collected, to thereby obtain a pure CNTF protein. (Hereinafter, unless otherwise stated, the thus-obtained CNTF protein will be referred to as "CNTF." Also, unless otherwise stated, in examples described below, the CNTF produced in this Reference Example was used as CNTF serving as an active ingredient in the skin external-use preparation of the present invention.)

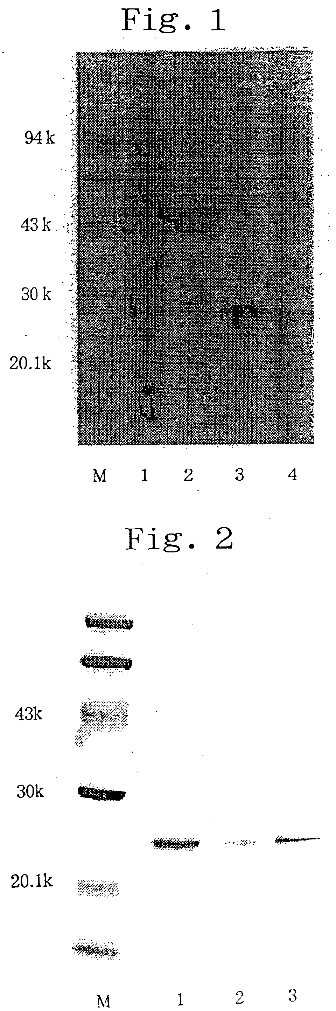

Fig. 1 is an electrophoresis profile showing the purification process of a GST-CNTF fusion protein from expression *E. coli*. (Lane 1 shows the results of crude water-soluble fractions of expression *E. coli*; lane 2 shows the results of purification of the crude fractions by use of a glutathione-sepharose column; lane 3 shows the results of treatment by use of thrombin; and lane 4 shows the results of removal of GST from a digested solution by use of a glutathione-sepharose column. Fig. 1 shows the process in which CNTF is purified through these steps. In Fig. 1, "k" represents kDa.)

### (2) Preparation of antibody to CNTF

The above-obtained CNTF was injected into a rabbit together with Freund's adjuvant, and this immunization procedure was repeated. Thereafter, serum was collected from the rabbit and purified by means of a customary method, to thereby obtain a polyclonal antibody. Specificity of the polyclonal antibody was confirmed by Western blotting of the above-obtained CNTF and an ischiatic nerve extract in which CNTF is expressed in a very large amount, by use of the antibody.

Fig. 2 is an electrophoresis profile showing the results of the Western blotting. (In Fig. 2, lanes 1, 2, and 3 show the results of Western blottings of CNTF, a human ischiatic nerve extract, and a rat ischiatic nerve extract, respectively. In Fig. 2, "k" represents kDa.) 〈Test Example〉 Study of utility of CNTF as an active ingredient in the skin external-use preparation of the present invention

### 1. Basic study

### (1) Study by use of keratinocyte

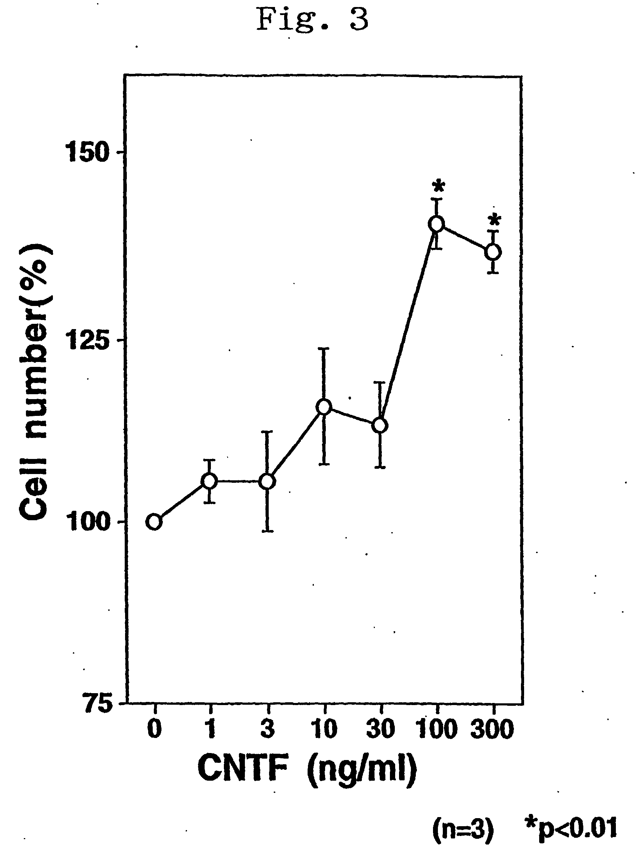

Human keratinocytes were added to Keratinocyte Basal Medium (Sigma), and the keratinocytes were inoculated in a collagen-coated 96-well plate (5000 cells/well). The keratinocytes were cultured at 37°C for 24 hours under 5% CO₂, and the above-obtained CNTFs of various concentrations were added to the well plate, and the keratinocytes were cultured at 37°C for 72 hours under 5% CO₂. Thereafter, the extent of proliferation of the keratinocytes in each well was determined by means of MTT assay (Sigma). The results are shown in Fig. 3 (horizontal axis: concentration of CNTF, vertical axis: the number of keratinocytes).

As is apparent from Fig. 3, keratinocytes significantly proliferate in wells to which CNTF (100 ng/ml, 300 ng/ml) has been added.

Keratinocyte is a basic cell in skin structure, which cell produces keratin, a protective protein specific to the skin, and ultimately forms a meshwork which is a fundamental constituent of the skin. As is apparent from the above test, CNTF exhibits excellent activation of proliferation of keratinocyte. Thus, when CNTF is incorporated as an active ingredient into the skin external-use preparation of the present invention, the preparation exhibits preventive and therapeutic effects on wounds and skin diseases or the like accompanied by ulcer, such as decubitus.

### (2) Study by tissue culture of human skin

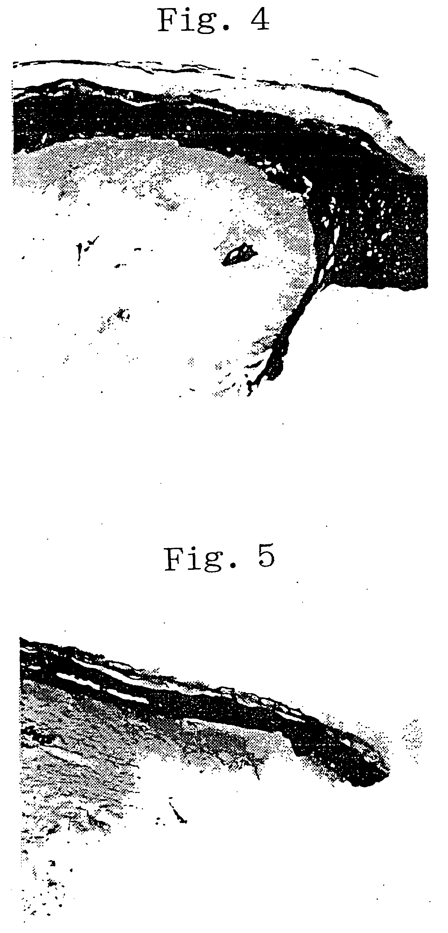

The above-obtained CNTF (75 ng/ml) was added to 0.25% bovine albumin-containing Iscove's Modified Dulbecco's Medium (IMEM), and a biopsy sample of skin obtained from an ALS patient (2 × 3 mm) was placed in the medium such that the exodermis surface faced downward, and was cultured at 37°C for seven days under 5% CO₂ in an incubator. The medium was replaced every 48 hours. After completion of culturing, the cultured product was formalin-fixed. After alcoholic dehydration, the product was embedded in paraffin, to thereby prepare a skin sample. Staining was performed by use of an anti-α-cytokeratin monoclonal antibody (ABC staining kit: product of Wako Pure Chemicals Industries, Ltd.). The results are shown in Figs. 4 and 5 as staining images. As is apparent from Figs. 4 and 5, remarkable proliferation of skin cells is observed in a skin sample containing CNTF (shown in Fig. 4) as compared with a skin sample containing no CNTF (only the above IMEM) (shown in Fig. 5), and elongation and thickening of epidermis are observed in a cut side of the skin.

As a result, CNTF was confirmed to indeed exhibit effects on thickening of human skin. Thus, from the results of the above-described two tests, including the test by use of keratinocyte, it is apparent that a skin external-use preparation containing CNTF as an active ingredient exhibits therapeutic effects on wounds and skin diseases or the like accompanied by ulcer, such as decubitus.

### 2. Study on wound-treatment effect (study on wound-treatment effect by use of rat)

A rat was anesthetized by use of ether, hair of the rat was removed by use of a shaver, and along the dorsomedial line, circles were marked by use of a circular punch having a diameter of 0.9 cm. Subsequently, the skin portions inside the circles were excised by use of scissors, to thereby artificially form wounds with loss of substance. One hour after skin excision, contours of the wound regions were traced onto a plastic sheet, and the following sample solutions (65 µl each) were administered dropwise to the wounds: (1) PBS (phosphate buffered saline); (2) human basic fibroblast growth factor (bFGF) (10 µg per cm² of skin area (hereinafter "µg per cm² of skin area" will be referred to as "µg/cm²")); and (3) CNTF (10 µg/cm²). Thereafter, the wounds were protected by use of a tape (BIOCLUSIVE (registered trademark): product of Johnson & Johnson). Three days, six days, nine days, and 12 days after initial administration of the sample solutions, in the same manner as described above, the solutions were administered dropwise to the wounds, and the areas of the portions with loss of substance were measured at every administration.

The results are shown in terms of percentage (%) of the area of the portion with loss of substance after treatment based on the area of the portion with loss of substance before starting the test, which is regarded as 100.

The area of wound with loss of substance was measured by use of an AREA-LINE METER (Super PLANIX β: product of Tamaya Keisoku System K.K.). In iso-dispersion of measurements of each administration group, when dispersion is considered to be uniform on the basis of the Bartlett method, a control group was compared with other administration groups by use of a modified Tukey's multiple comparison test. A statistic software STATISTICA (product of Design Technologies) was used for statistical processing of the test results.

The results of the test are shown in Table 1.

**Table 1**

| Sample | Area of wound with loss of substance (%) | | | |
|---|---|---|---|---|
| | 0 day | 6 days | 9 days | 12 days |
| PBS | 100 ± 0 | 46.92 ± 1.61 | 6.22 ± 0.79 | 0 ± 0 |
| bFGF 10 µg/cm² | 100 ± 0 | 37.75 ± 5.15 | 8.78 ± 2.94 | 0.30 ± 0.33 |
| CNTF 10 µg/cm² | 100 ± 0 | 32.25 ± 4.26* | 0.50 ± 0.33 | 0 ± 0 |
| In Table 1, each value refers to mean value ± S. E. of 5-6 rats. | | | | |

| | | | | |
|---|---|---|---|---|
| *: A significant difference exists in comparison with a PBS administration group (P < 0.05). | | | | |

As is apparent from Table 1, six days after creation of the loss-of-substance wounds, the area of the wound portion in a CNTF administration group (32.35 ± 4.26%) is reduced to some extent as compared with the case of a bFGF administration group (37.75 ± 5.15%), and is significantly decreased as compared with the case of a PBS administration group (46.92 ± 1.61%).

It is known that bFGF exhibits therapeutic effects on skin ulcer by promoting migration and proliferation of damaged cells and granulation and epithelialization through capillary vascularization (Okumura, Okuda, et al.: "Study on wound-curing effect of gene-recombinant-type human basic fibroblast growth factor (bFGF) - Usage and comparison between existing drugs and the active ingredient of bFGF in terms of action," *KISO to RINSHO*, 30, 2161-2174 (1996)).

As described above, the area of the wound with loss of substance in a CNTF administration group tends to decrease, and thus it is apparent that CNTF exhibits curative effects on skin wounds.

Therefore, it is apparent that when the skin external-use preparation of the present invention assumes the form of wound-treatment preparation, CNTF is a very excellent active ingredient.

### 3. Study on decubitus-treatment effect (study on high expression of CNTF on skin of ALS patients)

Biopsy skins of 10 ALS patients and 10 patients suffering other neuropathies were immediately formalin-fixed, and subjected to paraffin embedding, to thereby prepare skin samples. Subsequently, the skin samples were reacted with the above-described anti-CNTF-polyclonal antibody, and subjected to vital staining. Vital staining was performed by means of any method among HE staining, anti-CNTF antibody staining, and immunostaining by use of an anti-CNTF-antibody absorbed by CNTF (anti-CNTF antibody staining was performed by use of an ABC staining kit (product of Wako Pure Chemicals Industries, Ltd.))

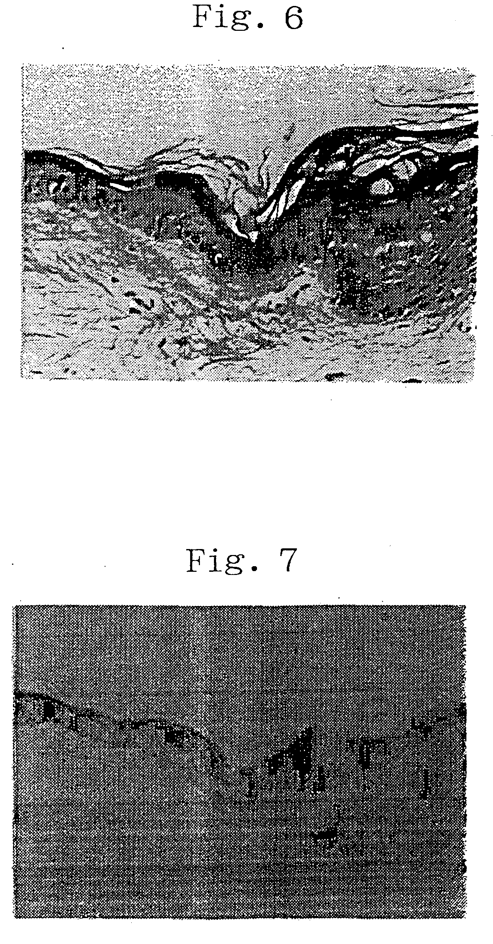

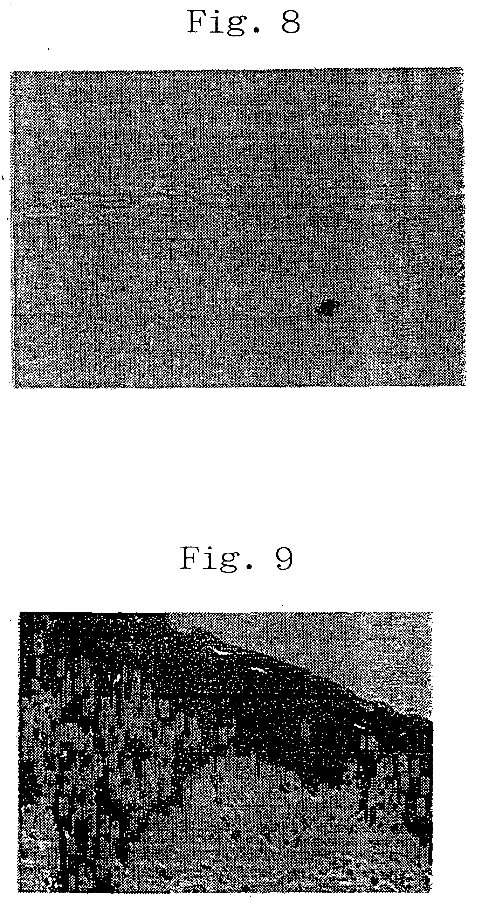

Skin samples from 10 ALS patients were intensely stained by means of anti-CNTF-antibody staining. Figs. 6 to 8 show vital staining images of skin samples of a patient who has suffered ALS over a long period of time (Fig. 6: HE staining, Fig. 7: anti-CNTF-antibody staining, Fig. 8: immunostaining by use of an anti-CNTF-antibody absorbed by CNTF).

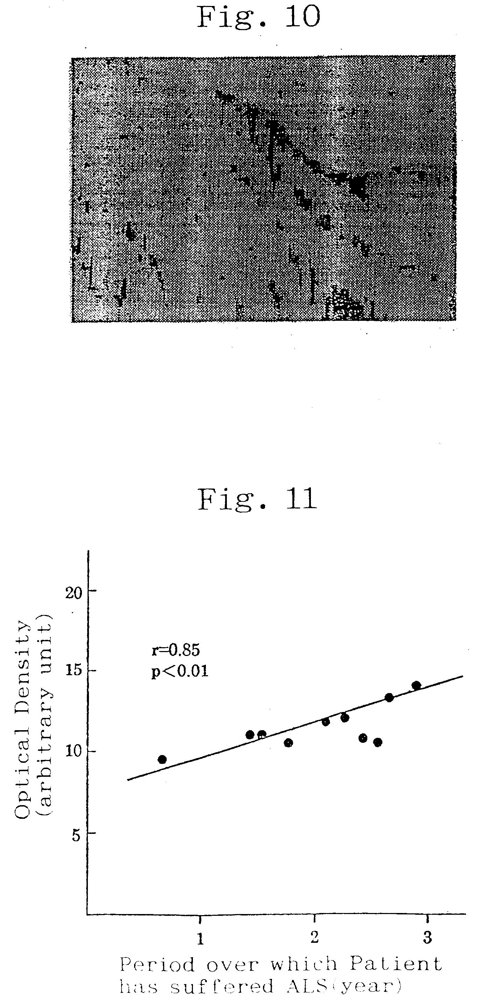

Figs. 9 and 10 show vital staining images of skin samples of a patient who has suffered ALS over a short period of time (Fig. 9: HE staining, Fig. 10: anti-CNTF-antibody staining).

In anti-CNTF-antibody staining, Fig. 7 shows that epidermal cells are strongly stained (the position of epidermal cells are confirmed by HE staining images shown in Figs. 6 and 9), but Fig. 10 shows that epidermal cells are weakly stained.

As a result, it is apparent that CNTF is expressed in the skin of an ALS patient, and the expression degree tends to be high when the patient has suffered ALS over a long period of time.

Fig. 11 is a graph showing a correlation between the period over which a patient has suffered ALS and the intensity of anti-CNTF-antibody staining. In Fig. 11, the horizontal axis corresponds to the period over which a patient has suffered ALS (years), and the vertical axis corresponds to the intensity of anti-CNTF-antibody staining (the intensity was determined by means of an image analysis system (Carl Zeiss, Germany)).

From analysis of the results shown in Fig. 11, it is also apparent that CNTF is expressed in the skin of an ALS patient, and the expression degree tends to be high when the patient has suffered ALS over a long period of time.

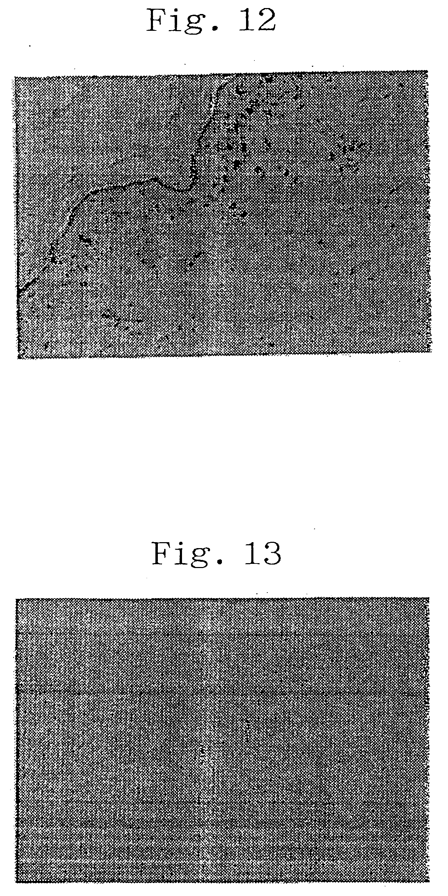

Fig. 12 is an HE staining image of the skin sample of a patient suffering a neuropathy other than ALS, and Fig. 13 is an anti-CNTF-antibody immunostaining image of the skin sample of a patient suffering a neuropathy other than ALS. As is apparent from this immunostaining image, the skin sample of a patient suffering a neuropathy other than ALS is not stained by CNTF-antibody staining.

As a result, it is apparent that expression of CNTF in the skin is not a phenomenon simply caused by a neuropathy, but a specific phenomenon observed in an ALS patient.

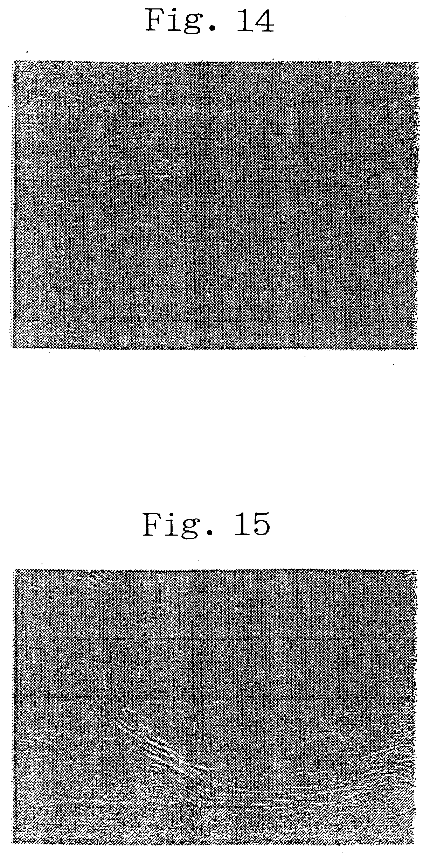

Fig. 14 is an immunohistoimage of the skin sample of an ALS patient who has been confirmed to have decubitus, which is a rare occurrence, obtained by use of anti-CNTF antibody. Fig. 15 is a vital staining image of the skin sample of a patient suffering a neuropathy other than ALS (Parkinson's disease), who has been confirmed to have decubitus, obtained by use of anti-CNTF antibody. In both these immunotissue images, no staining was observed.

As a result, it is apparent that whether or not decubitus is confirmed fundamentally depends not on morbidity to ALS but on expression of CNTF in the surface of the skin.

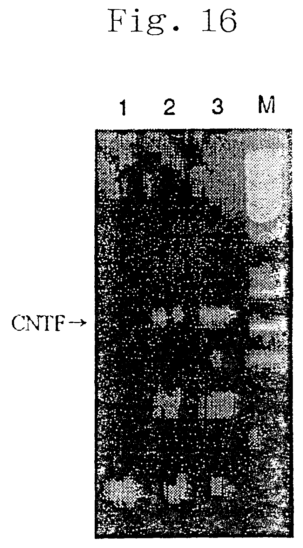

Fig. 16 is an electrophoresis profile showing the results of studies as to whether or not CNTF is highly expressed in the skin of an ALS patient, by reference to mRNA serving as an index.

Fig. 16 is an electrophoresis profile showing the results of detection of mRNA of CNTF. In order to detect mRNA of CNTF, biopsy skins of two ALS patients and a patient suffering a neuropathy other than ALS were immediately frozen, and all RNA were extracted from the frozen biopsy skins by means of an SV total RNA Isolation System (Promega, USA). Subsequently, all the thus-extracted RNA were subjected to the RT-PCR method employing a primer specific to CNTF (lanes 2 and 3: electrophoresis profiles of mRNA of the skin of ALS patients, and lane 1: an electrophoresis profile of mRNA of the skin of a patient suffering a neuropathy other than ALS).

As is apparent from Fig. 16, CNTF-mRNA is observed in the skin of two ALS patients, but is not observed in the skin of a patient suffering a neuropathy other than ALS. Thus, it is apparent that CNTF is considerably expressed in dermal cells of the ALS patients.

As a result, CNTF is confirmed to exhibit protective effects on decubitus.

### 〈Formulation Example〉 Preparation of CNTF-containing macrogol ointment

Macrogol 1000 (500 g) and macrogol 400 (500 g) (total: 1,000 g) were warmed to 65°C and melted in a hot water bath, and stirred well until solidification, to thereby obtain a base material. Independently, CNTF (1 g) was dissolved in sterile water (10 ml). The resultant solution was blended with the above-obtained base material, to thereby obtain an ointment of interest.

### Industrial Applicability

As described above, the present invention provides a skin external-use preparation exhibiting therapeutic effects on wounds and skin diseases or the like accompanied by ulcer, such as decubitus.

## Claims

1. A skin external-use preparation comprising a ciliary neurotrophic factor as an active ingredient.

2. A skin external-use preparation according to claim 1, wherein the ciliary neurotrophic factor is of human derivation.

3. A skin external-use preparation according to claim 1 or 2, wherein the preparation is a decubitus-therapeutic preparation.

4. A skin external-use preparation according to claim 1 or 2, wherein the preparation is a wound-therapeutic preparation.
